(19) Europäisches Patentamt
European Patent Office
Office européen des brevets

(11) Veröffentlichungsnummer: **0 004 574**
**B1**

# (12) EUROPÄISCHE PATENTSCHRIFT

(45) Veröffentlichungstag der Patentschrift: **30.12.81**

(51) Int. Cl.³: **C 07 C 143/78,** C 07 C 143/822, C 07 D 295/12, C 07 D 295/22, G 03 C 1/58

(21) Anmeldenummer: **79100723.0**

(22) Anmeldetag: **12.03.79**

## (54) 2-Hydroxy-3-naphthoesäureamide und deren Verwendung als Kupplungskomponenten in Diazotypiematerialien.

(30) Priorität: **18.03.78 DE 2811981**

(43) Veröffentlichungstag der Anmeldung: **17.10.79 Patentblatt 79/21**

(45) Bekanntmachung des Hinweises auf die Patenterteilung: **30.12.81 Patentblatt 81/52**

(84) Benannte Vertragsstaaten: **BE CH DE FR GB IT NL SE**

(56) Entgegenhaltungen:
**DE-A-1 572 078**
**DE-A-2 414 498**
**FR-A- 953 727**

(73) Patentinhaber: **HOECHST AKTIENGESELLSCHAFT, KALLE, Patentabteilung Postfach 3540, D-6200 Wiesbaden 1 (DE)**

(72) Erfinder: **Scheler, Siegfried, Dr., Stormstrasse 5, D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# 2-Hydroxy-3-naphthoesäureamide und deren Verwendung als Kupplungskomponenten in Diazotypiematerialien

Die Erfindung bezieht sich auf 2-Hydroxy-3-naphthoesäureamide, welche in 6-Stellung durch eine Sulfonamidgruppe substituiert sind.

Bekannte 2-Hydroxy-3-naphthoesäureamide, die in 6-Stellung nicht substituiert sind (DE-AS 1178704) oder durch Halogen oder eine niedere Alkoxygruppe (DE-AS 1572078) substituiert sind, werden schon seit längerer Zeit als blau kopierende Kuppler in Diazotypiematerialien eingesetzt. In Kombination mit gelb bzw. braun kuppelnden Komponenten können sie auch für schwarz kopierende Diazotypiematerialien eingesetzt werden.

Es ist ferner bekannt (DE-AS 1240738), 1-Hydroxy-naphthalin-8-sulfonamide in Diazotypiematerialien für die Trockenentwicklung als kupplungsfähige Verbindung einzusetzen, wodurch tiefblau-grünstichige Kopien erhalten werden.

Ein Nachteil dieser bekannten Kuppler ist, dass ihre Azofarbstoffe, die bei der Kupplung mit den in der Diazotypie üblichen Benzoldiazoniumverbindungen gebildet werden, nur eine mässige, teilweise sogar anwendungstechnisch völlig unzureichende Lichtechtheit aufweisen. Die bekannte leichte Ausbleichbarkeit von Azofarbstoffen bei der Lichteinwirkung führt bei der technischen Anwendung von Diazokopien zu erheblichen Problemen. Die schlechte Lichtechtheit von Azofarbstoffen ist besonders dann von Nachteil, wenn eine Diazokopie, zum Beispiel eine technische Zeichnung, längere Zeit dem Sonnenlicht oder ein sogenanntes «Diazozwischenoriginal», das für die Herstellung von weiteren Diazokopien verwendet werden soll, längere Zeit dem aktinischen Licht handelsüblicher Lichtpausgeräte ausgesetzt wird.

Ganz besonders hohe Anforderungen an die Lichtechtheit von Azofarbstoffen werden aber an Diazomikrofilmkopien gestellt, die in der aktiven Mikrofilmanwendung als sogenannte Lese- oder Arbeits-Kopien eingesetzt werden. Solche Diazomikrofilmkopien, die zur Auswertung ihrer Informationen in Lesegeräten oder Leserückvergrösserungsgeräten eingesetzt werden, sind durch deren Lichtquellen einer besonders starken Strahlungsbelastung – Wärmestrahlung, sichtbares Licht, UV-Strahlung – ausgesetzt. Es kommt häufig vor, dass die Farbstoffbezirke einer Diazomikrofilmkopie, die längere Zeit den in den handelsüblichen Lesegeräten verwendeten Lampen ausgesetzt sind, so stark ausbleichen und an Konstrast verlieren, dass die Information nur noch schwer erkennbar oder sogar völlig gelöscht ist.

Es ist auch bekannt, das Ausbleichen oder die Vergilbungsneigung einer Diazokopie dadurch zu verhindern, dass man zur Stabilisierung der darin enthaltenen Azofarbstoffe der lichtempfindlichen Schicht Harnstoff und Acrylamide (DE-AS 1572104) oder sogenannte sterisch gehinderte Phenole, die in 2- und 4-Stellung Substituenten tragen (DE-OS 1772981), zusetzt. Es hat sich jedoch gezeigt, dass durch beide Massnahmen in der praktischen Anwendung nur graduelle Verbesserungen zu erzielen sind.

Es war deshalb Aufgabe der Erfindung, Verbindungen zur Verfügung zu stellen, die als blau kuppelnde Komponenten zusammen mit den in der Diazotypie üblichen Benzoldiazoniumverbindungen Azofarbstoffe bilden, die eine bessere Lichtechtheit gegenüber den bekannten besitzen.

Die gestellte Aufgabe wird gelöst durch 2-Hydroxy-3-naphthoesäureamide der allgemeinen Formel

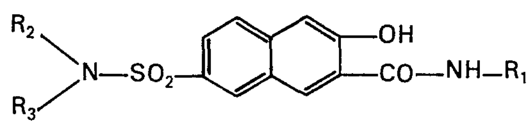

worin $R_1$ Wasserstoff oder Alkyl, Cycloalkyl, Aralkyl, Aryl, welche durch Hydroxyl, Alkyl, Halogenalkyl, Alkoxyl, Acyl, Halogen, Dialkylamino oder einen heterocyclischen Rest substituiert sein können, und

$R_2$ und $R_3$ gleich oder verschieden sind und Wasserstoff oder Alkyl, Cycloalkyl, Aralkyl, Aryl, welche durch Hydroxyl, Niederalkyl, Alkoxyl, Acyl oder Halogen substituiert sein können oder zusammen mit dem Stickstoffatom, an das sie gebunden sind, einen heterocyclischen Rest, der durch Alkyl substituiert sein kann,

bedeuten. Hierbei erweisen sich 2-Hydroxy-3-naphthoesäureamide der allgemeinen Formel als günstig, bei denen $R_1$ Wasserstoff, Alkyl mit bis zu 4 Kohlenstoffatomen, Cycloalkyl mit bis zu 12 Kohlenstoffatomen, Aralkyl mit bis zu 10 Kohlenstoffatomen, oder Aryl mit bis zu 10 Kohlenstoffatomen, welche durch Hydroxyl, gegebenenfalls halogensubstituiertes Alkyl, Alkoxyl, Acyl, Halogen, durch gegebenenfalls alkylsubstituierte Aminogruppen oder durch einen heterocyclischen Rest substituiert sein können, $R_2$ und $R_3$ gleich oder verschieden sind, und Wasserstoff, Alkyl mit bis zu 6 Kohlenstoffatomen, Cycloalkyl mit bis zu 6 Kohlenstoffatomen, Aralkyl mit bis zu 10 Kohlenstoffatomen oder Aryl mit bis zu 10 Kohlenstoffatomen, welche durch Hydroxyl, niederes Alkyl, Alkoxyl oder Acyl, Halogen substituiert sein können, oder zusammen mit dem Stickstoffatom, an das sie gebunden sind, gegebenenfalls Alkyl-substituiertes Pyrrolidin, Piperidin, Piperazin, Thiomorpholin, Morpholin oder Hexamethylenimin bedeuten. Bevorzugt werden solche Verbindungen, in denen $R_1$ und $R_2$ gleiches oder verschiedenes Aryl, welches durch Hydroxyl, Niederalkyl, Alkoxyl oder Halogen substituiert sein kann, und $R_3$ Wasserstoff bedeuten. Sehr gute Ergebnisse erzielt man, wenn Verbindungen der allgemeinen Formel eingesetzt werden, in denen $R_1$ und $R_2$ Phenyl darstellen, das durch mindestens ein Hydroxyl, niederes Alkyl oder Alkoxyl

oder Halogen substituiert sein kann, und $R_3$ Wasserstoff bedeutet. Letztere setzt man in der Diazotypie besonders dort ein, wo Sensibilisierungsformulierungen auf Lösungsmittelbasis verwendet werden.

Mit den erfindungsgemässen 2-Hydroxy-3-naphthoesäureamiden wird eine Kupplerkomponente für die Diazotypie zur Verfügung gestellt, die es erlaubt, Diazotypiekopien, insbesondere Diazomikrofilmkopien zu schaffen, welche den hohen Forderungen an Lichtechtheit gerecht werden und die auch bei erhöhter Strahlungsbelastung Farbe und Kontrast wahren.

Für den Einsatz von wässrigen Sensibilisierungsformulierungen sind besonders solche Verbindungen der allgemeinen Formel bevorzugt, bei denen $R_1$ und/oder $R_2$ durch wasserlöslich machende Gruppen, z.B. Hydroxyl- oder Alkoxylgruppen, substituiert sind. Ganz besonders bevorzugt sind solche Verbindungen der allgemeinen Formel, bei denen $R_1$ und/oder $R_2$ durch mindestens einen basischen Rest substituiert sind, der als solcher, als Salz, als Schwermetall-Komplexsalz oder als quaternäres Ammoniumsalz vorliegen kann. Die Salzbildung am basischen Rest kann in bekannter Weise mit jeder Säure erfolgen, die beständige Aminsalze bildet. Hierbei werden die Hydrochloride bevorzugt. Als komplexe Schwermetall-Aminsalze sind die Zinkchlorid-Komplexsalze bevorzugt. Ist der basische Rest quaterniert, so ist der vierte organische Rest vorzugsweise eine niedere Alkylgruppe. Der basische Rest kann auch ein heterocyclischer Rest sein. Bevorzugt sind basische Heterocyclen mit 5–8 Ringgliedern, wie zum Beispiel Pyrrolidin, Piperidin, Piperazin, Thiomorpholin, Hexamethylenimin, besonders jedoch Morpholin.

Beispiele für erfindungsgemässe 2-Hydroxy-3-naphthoesäureamide, welche vorzugsweise als Kupplungskomponenten in Diazotypiematerialien verwendet werden, sind zusammen mit ihren Schmelz- bzw. Zersetzungspunkten der beigefügten Formelsammlung zu entnehmen.

Die Herstellung der erfindungsgemässen 2-Hydroxy-3-naphthoesäureamide geschieht nach folgenden Verfahren:

Verfahren A

Die durch Alkyl- bzw. Aralkylreste $R_1$ substituierten 2-Hydroxy-3-naphthoesäureamid-6-sulfonsäureamide erhält man auf folgendem Wege:

2-Hydroxy-3-naphthoesäure-6-sulfonsäure (K-Salz) wird in bekannter Weise in Methanol/Schwefelsäure conc. zu 2-Hydroxy-3-naphthoesäure-methylester-6-sulfonsäure (K-Salz) verestert. Anschliessend wird die phenolische Hydroxylgruppe nach Schotten-Baumann in wässriger Sodalösung durch Umsetzung mit Benzolsulfonsäurechlorid verschlossen und dann die Sulfonsäuregruppe durch Reaktion mit Phosphoroxidchlorid in die Sulfonsäurechloridgruppe übergeführt. Das erhaltene 2-Benzolsulfonyloxy-3-naphthoesäuremethylester-6-sulfonsäurechlorid schmilzt bei 156–157 °C und kann nach bekannten Verfahren mit den durch die Reste $R_2$ und $R_3$ substituierten Aminbasen zu den entsprechenden Sulfonsäureamiden umgesetzt werden. Aus den 2-Benzolsulfonyloxy-3-naphthoesäuremethylester-6-sulfonsäureamiden erhält man durch Aminolyse der Methylester mit Ammoniak bzw. primären aliphatischen Aminen analog der in der holländischen Patentschrift 65 14 065 für die Herstellung der Verbindung Nr. 12 beschriebenen Verfahrensweise die erfindungsgemässen 2-Hydroxy-3-naphthoesäureamid-6-sulfonsäureamide. Bei der Aminolysereaktion wird gleichzeitig die Benzolsulfonyl-Schutzgruppe abgespalten. Die auf diesem Reaktionswege synthetisierten Verbindungen sind in der angefügten Formelsammlung mit A bezeichnet.

Verfahren B

Zur Herstellung der durch Aryl- bzw. Cycloalkylreste $R_1$ substituierten 2-Hydroxy-3-naphthoesäureamid-6-sulfonsäureamide werden die nach Verfahren A hergestellten 2-Benzolsulfonyloxy-3-naphthoesäuremethylester-6-sulfonsäureamide kurzzeitig in wässrig-alkalischer Lösung erwärmt, wobei Verseifung der Carbonsäuremethylester und gleichzeitig Abspaltung der Benzolsulfonyl-Schutzgruppe erfolgt. Anschliessend wird aus den 2-Hydroxy-3-naphthoesäure-6-sulfonsäureamiden nach bekannten Verfahren mit Thionylchlorid in einem indifferenten Lösungsmittel analog der Herstellung der Carbonsäurechloride aus DE-AS 15 72 078, Beispiel 1, das Carbonsäurechlorid hergestellt und dieses, ohne es zu isolieren, in einer «Eintopfreaktion» mit den durch die Aryl bzw. Cycloalkylreste $R_1$ substituierten primären Aminen zu den entsprechenden Carbonsäureamiden umgesetzt.

Die auf diesem Reaktionswege synthetisierten Verbindungen sind in der angefügten Formelsammlung mit B bezeichnet.

Als Trägermaterial für Diazotypieschichten ist jede übliche Unterlage geeignet wie beschichtete oder unbeschichtete, opake oder transparente Papiere, Textilien oder Kunststoffolien. Besonders geeignete Kunststoffolien sind solche aus Celluloseester, wie Cellulose 2½- bzw. -triacetat, aus Polyester, wie Polyäthylenterephthalat, aus Vinylpolymeren, wie Polyvinylacetat oder Polyvinylstyrol.

Diazoverbindungen, die mit den erfindungsgemässen Kupplungskomponenten zur Erzeugung blauer Farbtöne verwendet werden, sind bekannt. Sie sind überwiegend Derivate des einseitig diazotierten p-Phenylendiamins mit mindestens einem Substituenten, z.B. Alkyl- oder Hydroxyalkyl, in der Aminogruppe. Bevorzugt sind solche Diazoverbindungen vom einseitig diazotierten p-Phenylendiamintyp, die in 2- und 5-Stellung durch einen Alkoxyrest substituiert sind und bei denen die Substituenten am Aminostickstoffatom, an das sie gebunden sind, zu einem heterocyclischen Ringsystem, zum Beispiel ein Morpholinringsystem, verknüpft sind.

Die erfindungsgemässen 2-Hydroxy-3-naphthoesäureamide ergeben zusammen mit den Diazoverbindungen kräftige neutral blaue bis grünstichig blaue Azofarbstoffe. Im Vergleich zu den bekannten blauen Azofarbstoffen zeichnen sie sich durch erheblich bessere Lichtechtheit aus.

Vorteilhaft gegenüber den bekannten 2-Hydroxy-3-naphthoesäureamiden, die in der 6-Stellung z.B. durch die Methoxygruppe oder durch ein Bromatom substituiert sind, ist ausserdem die sehr geringe Eigenfärbung dieser neuen Verbindung. Der Bildhintergrund von Diazokopien, bei denen die erfindungsgemässen Verbindungen als Kupplungskomponenten in der Kopierschicht enthalten sind, ist deshalb besser als der, bei dem die bekannten verwendet werden.

Ein weiterer Vorteil der vorliegenden 2-Hydroxy-3-naphthoesäureamide gegenüber den bereits bekannten ist ihre geringere Kupplungsgeschwindigkeit. Diese führt in Kombination mit den bei Zweikomponentenmaterialien bevorzugt verwendeten Diazoverbindungen vom p-Phenylendiamintyp zu einer deutlich verbesserten Halbarkeit des mit diesen neuen Kupplungskomponenten hergestellten unbelichteten Diazotypiematerials; sowohl unter normalen als auch unter feuchten klimatischen Bedingungen. Aufgrund der verminderten Kupplungsgeschwindigkeit dieser neuen Kupplungskomponenten können für die Herstellung von Zweikomponenten-Diazotypiematerialien auch schneller kuppelnde Diazoverbindungen eingesetzt werden, die üblicherweise sonst nur in Einkomponenten-Diazotypiematerialien verwendet werden, z.B. Diazoverbindungen, die in p-Stellung zur Diazoniumgruppe durch einen Mercapto-, Phenyl- oder Acylamino-Rest substiuiert sind, ohne die Lagerfähigkeit des Materials für praktische Anwendungen zu beeinflussen.

Die erfindungsgemässen 2-Hydroxy-3-naphthoesäureamide als Kupplungskomponenten können auch im Gemisch untereinander oder zur Erzielung anderer gewünschter Farbtöne im Gemisch mit Kupplungskomponenten anderer Farbtöne eingesetzt werden.

Die vorzugsweise für Zweikomponenten-Diazotypiematerialien verwendeten erfindungsgemässen Kupplungskomponenten werden zusammen mit der Diazokomponente in bekannter Weise aus wässriger, wässrig-alkoholischer oder aus rein organischer Lösung auf den geeigneten Träger aufgebracht.

Werden Kunststoffolien als Trägermaterialien verwendet, hat es sich als vorteilhaft erwiesen, die Diazotypiebestandteile aus einem organischen Medium, das ein filmbildendes Bindemittel enthält, in Form einer Lösung auf den Schichtträger aufzubringen, Die Konzentration der farbstoffbildenden Komponenten im Bindemittel kann variieren zwischen 15 und 30 Gewichtsteilen der farbstoffbildenden Komponenten pro 100 Gewichtsteile Bindemittel.

Beispiele für geeignete Bindemittel sind die verschiedensten polymeren Substanzen, beispielsweise Celluloseäther, wie Äthylcellulose, sowie Celluloseester, wie Celluloseacetat, -triacetat, -acetopropionat, -butyrat und -acetobutyrat, Vinylpolymerisate, wie Polyvinylacet, Polyvinylidenchlorid, Vinylchlorid/Vinylacetat-Mischpolymerisate, Poly-(methyl-methacrylat)-Mischpolymerisate von Alkylacrylaten und Acrylsäure, sowie weitere Polymerisate, z.B. Polyphenylenoxid und Äthylenglykol/Isophthalsäure/Terephthalsäure-Terpolymerisate.

Die lichtempfindliche Schicht kann ausser den farbstoffbildenden Komponenten zusätzlich noch saure Stabilisatoren wie Salzsäure, Borsäure, Zitronensäure, Weinsäure, Ameisensäure und 5-Sulfosalicylsäure und andere übliche Diazotypiehilfsstoffe enthalten.

Häufig enthalten die Sensibilisierungslösungen auch anorganische Salze wie Zinkchlorid und Ammonsulfat zur Kontraststeigerung und Zusätze als Lösungsvermittler und Kupplungsbeschleuniger, beispielsweise Harnstoff oder Thioharnstoff oder auch organische Lösungsmittel, wie Aceton, Methyläthylketon, Äthylenglykol, Äthylenglykolmonomethyläther, Glycerin, Glycerindi- und Glycerintriacetat u.a.

Weiterhin können der lichtempfindlichen Schicht Antivergilbmittel und Weichmacher zugesetzt werden. Auch Farbstoffe in kleiner Konzentration zur Anfärbung der ausbelichteten Bereiche der Kopie können in der Diazotypieschicht anwesend sein.

Die folgende Beispiele sollen die Erfindung näher erläutern.

Erklärungen zu den Beispielen:

A. Der für die Herstellung der Diazotypiematerialien in den Beispielen 1–5 verwendete Basislack enthält 7,5 Gewichtprozent Celluloseacetopropionat, gelöst in einem Lösungsmittelgemisch aus Aceton, Methanol, n-Butanol und Glykolmonomethyläther.

B. Die optischen Dichten der Volltonmuster der Beispiele 1–5 werden mit einem MACBETH Quantalog-Densitometer gemessen.

Für die Dichtemessung im visuellen Spektralbereich wird ein Filter, das im Bereich von 510 bis 580 nm wirksam ist, für die Dichtemessung im ultravioletten Spektralbereich ein Filter, das im Bereich von 310 bis 410 nm wirksam ist, verwendet.

C. Zur Prüfung und Beurteilung der Lichtechtheit der Diazotypiefarbstoffe der Volltonmuster der Beispiele 1–5 werden folgende Geräte verwendet:

Ein übliches Lesegerät mit Glühlampe 15 V/150 Watt und mit einem Objektiv für 24fache Vergrösserung bei maximaler Hellschaltung.

Ein Leuchtstoffröhren-Testgerät mit 5 Leuchtstoffröhren Philips TLAD 15 W/05. Die spektrale Zusammensetzung der Strahlungsemission der Leuchtstoffröhren entspricht etwa der des Sonnenlichtes.

D. Die Entwicklung der Volltonmuster der Beispiele 1–5 erfolgt konventionell mit feuchtem Ammoniakgas und wird im Entwicklerteil eines handelsüblichen Mikroplanfilm-Dupliziergerätes durchgeführt.

Die bildmässige Belichtung der Diazotypienmaterialien des Beispiels 6 und die anschliessende Entwicklung der latenten Diazotypienkopien

erfolgt in einem handelsüblichen Lichtpausgerät.

Beispiel 1

Ausgehend von jeweils 100 ml Basislack werden 4 verschiedene Beschichtungslösungen der folgenden Zusammensetzung hergestellt:
360 mg Sulfosalicylsäure
420 mg 2,5-Dibutoxy-4-morpholinobenzol-diazoniumfluoborat
$1 \times 10^{-3}$ Mol Blaukuppler
wobei als Blaukuppler verwendet wurden:
1.: 2-Hydroxy-3-naphthoesäure-N(2-methylphenyl)-amid
2.: 6-Methoxy-2-hydroxy-3-naphthoesäure-N(2-methylphenyl)-amid
3.: 6-Brom-2-hydroxy-3-naphthoesäure-N(2-methylphenyl)-amid
4.: 2-Hydroxy-3-naphthoesäure-N(2-methylphenyl)-amid-6- sulfonsäure-N(phenyl)-amid (Nr. 16 der Formelsammlung).

Jede der Lösungen wurde auf eine mit einer Haftschicht versehene Polyäthylenterephthalatfolie von 100 µm Dicke derart aufgetragen, dass nach der Trocknung im Umlufttrockenschrank bei 60–70 °C die erhaltenen verschiedenen Diazotypienmaterialien zu einer optischen visuellen Dichte von etwa 2 entwickelt werden konnten.

Die hergestellten Diazotypiematerialien wurden, entsprechend der verwendeten Sensibilisierungslösungen, fortlaufend von 1–4 numeriert.

Das Diazotypiematerial 1 entwickelte zu einem rotstichig blauen Azofarbstoff, die Diazotpyiematerialien 2–4 zu grünstichig blauen Azofarbstoffen.

Um sicherzustellen, dass die Kupplung zum Azofarbstoff in der lichtempfindlichen Schicht vollständig abgelaufen war, wurden die 4 verschiedenen Diazotypiematerialien jeweils zweimal entwickelt.

Anschliessend wurden die zum Vollton entwickelten Diazotypiematerialien 12 Stunden lang dem diffusen Tageslicht ausgesetzt.

Von jedem der so behandelten Volltonmuster wurde ein Streifen von 0,5 cm × 5 cm Fläche abgeschnitten und dieser durch eine kreisförmige Messfläche von ca. 0,3 $cm^2$ markiert. In jeder Messfläche wurde die optische Dichte (D) im grünen und ultravioletten Spektralbereich bestimmt. Dann wurden die markierten Farbstoffstreifen der spektralen Emission der Lichtquellen eines Lesegerätes gleichzeitig ausgesetzt. Nach jeweils 2stündiger Bestrahlungszeit wurden von den Volltonmustern erneut die optischen Dichten ($D_{END}$) bestimmt und diese mit den Anfangsdichten ($D_{ANF}$) verglichen. Die Bestrahlung der Volltonmuster im Lesegerät wurde solange fortgesetzt, bis bei einer Probe die optische visuelle Dichte auf ≦ 0,5 abgesunken war.

Zur Beurteilung der Lichtechtheit der Azofarbstoffe der Volltonmuster 1–4 wurden ihre optischen Dichten zu Beginn (ANF) des Bestrahlungsversuches und nach 18 Stunden Standzeit (END) im Lesegerät in der folgenden Tabelle 1 zusammengestellt.

Tabelle 1

| Probe Nr. | visuelle Dichte $D_{ANF}$ | $D_{END}$ | Rest dichte % | UV-Dichte $D_{ANF}$ | $D_{END}$ | Rest-dichte % |
|---|---|---|---|---|---|---|
| 1 | 2,29 | 0,44 | 19 | 0,83 | 0,36 | 43 |
| 2 | 2,01 | 0,42 | 21 | 1,04 | 0,54 | 52 |
| 3 | 2,12 | 0,48 | 23 | 0,95 | 0,42 | 44 |
| 4 | 2,11 | 1,64 | 78 | 0,95 | 0,80 | 84 |

Ein Vergleich der optischen Dichtewerte zeigt sehr eindrucksvoll die überragende Lichtechtheit des Volltonmusters 4 sowohl im visuellen als auch im ultravioletten Spektralbereich.

Während der Azofarbstoff des Volltonmusters 4 nach 18 Stunden Standzeit im Lesegerät noch 78% seiner ursprünglichen visuellen Dichte aufweist, sind die Azofarbstoffe der Vergleichsmuster 1–3 bereits auf ca. 20% ihrer ursprünglichen visuellen Dichte abgefallen.

In Abb. 1 und Abb. 2 sind die gemessenen optischen Dichten der Volltonmuster 1–4 als Funktion der Bestrahlungszeit im Lesegerät in den Bereichen von 510 bis 580 nm (Abb. 1) und von 310 bis 410 nm (Abb. 2) graphisch dargestellt.

Während der Farbton des Volltonmusters 4 sich nach 18 Stunden Standzeit im Lesegerät nicht verändert, werden die Farbtöne der Volltonmuster 1–3 von anfangs Blau nach Rot verschoben.

Von den zum Vollton entwickelten und 12 Stunden in diffusem Tageslicht offen gelagerten Mustern 1–4 wurde jeweils ein zweiter Streifen von 3 cm × 5 cm Fläche abgeschnitten und durch eine kreisförmige Messfläche von ca. 0,3 $cm^2$ markiert. In der Messfläche wurden die optischen Dichten ($D_{ANF}$) im grünen und ultravioletten Spektralbereich bestimmt und dann die Proben in einem geschlossenen Metallbehälter 96 Stunden der spektralen Emission von 5 Leuchtstoffröhren ausgesetzt. Anschliessend wurden in den Messflächen erneut die optischen Dichten ($D_{END}$) bestimmt.

Das Ergebnis ist in der folgenden Tabelle zusammengestellt.

Tabelle 2

| Probe Nr. | visuelle Dichte $D_{ANF}$ | $D_{END}$ | Rest-dichte % | UV-Dichte $D_{ANF}$ | $D_{END}$ | Rest-dichte % |
|---|---|---|---|---|---|---|
| 1 | 2,20 | 1,76 | 80 | 0,81 | 0,72 | 89 |
| 2 | 1,75 | 1,50 | 86 | 0,91 | 0,85 | 93 |
| 3 | 1,93 | 1,69 | 87 | 0,88 | 0,81 | 91 |
| 4 | 2,07 | 1,95 | 94 | 0,95 | 0,90 | 95 |

Ein Vergleich der Dichtewerte zeigt, dass durch die spektrale Emission der Leuchtstoffröhren der Azofarbstoff des Volltonmusters 4 am wenigsten in seiner optischen Dichte verändert wurde.

Beispiel 2

Ausgehend von jeweils 100 ml Basislack werden 5 verschiedene Beschichtungslösungen der folgenden Zusammensetzung hergestellt:

360 mg Sulfosalicylsäure
420 mg 2,5-Dibutoxy-4-morpholinobenzoldiazoniumfluoborat
$1 \times 10^{-3}$ Mol Blaukuppler
wobei als Blaukuppler verwendet wurden:
5.: 2-Hydroxy-3-naphthoesäure-N(3-morpholinopropyl)-amid
6.: 6-Methoxy-2-hydroxy-3-naphthoesäure-N(3-morpholinopropyl)-amid
7.: 6-Brom-2-hydroxy-3-naphthoesäure-N(3-morpholinopropyl)-amid
8.: 8-Hydroxy-5-methyl-naphthalinsulfonsäureamid-(1)
9.: 2-Hydroxy-3-naphthoesäure-N(3-morpholinopropyl)-amid-6-sulfonsäure-N(phenyl)-amid (Nr. 3 der Formelsammlung)

Von jeder Lösung wurde wie im Beispiel 1 beschrieben, ein Diazotypiematerial mit laufender Numerierung hergestellt. Anschliessend wurde jedes Muster wie in Beispiel 1 zum Vollton entwickelt, und die so erhaltenen Volltonmuster wurden der Lichtechtheitsprüfung unterzogen.

Das Diazotypiematerial 5 entwickelte zu einem rotstichig blauen Farbton, die Diazotypiematerialien 6–9 zu grünstichig blauen Farbtönen.

Zur Beurteilung der Lichtechtheit der Azofarbstoffe der Volltonmuster 5–9 sind ihre optischen Dichten (D) zu Beginn (ANF) des Bestrahlungsversuches und nach 10 Stunden Standzeit (END) im Lesegerät der folgenden Tabelle zusammengestellt.

Tabelle 3

| Probe Nr. | visuelle Dichte $D_{ANF}$ | $D_{END}$ | Rest-dichte % | UV-Dichte $D_{ANF}$ | $D_{END}$ | Rest-dichte % |
|---|---|---|---|---|---|---|
| 5 | 2,20 | 0,45 | 20 | 0,73 | 0,32 | 44 |
| 6 | 1,87 | 0,28 | 15 | 0,87 | 0,39 | 45 |
| 7 | 2,26 | 0,24 | 11 | 0,87 | 0,27 | 31 |
| 8 | 1,92 | 0,63 | 33 | 0,80 | 0,44 | 55 |
| 9 | 2,07 | 1,38 | 67 | 0,82 | 0,64 | 78 |

Die gemessenen Dichtewerte zeigen, dass der Azofarbstoff des Volltonmusters 9 im Lesegerät erheblich lichtstabiler ist als die Azofarbstoffe der Volltonmuster 5–8. Während der Azofarbstoff des Volltonmusters 9 nach 10 Stunden Standzeit im Lesegerät noch 67% seiner ursprünglichen visuellen Dichte aufweist, sind die Azofarbstoffe der Vergleichsmuster 5–8 nahezu vollständig zerstört, wobei Probe 8 eine Standzeit von nur 6 Stunden hatte.

Der Farbton des Volltonmusters 9 bleibt nach 10 Stunden Standzeit im Lesegerät erhalten, die Farbtöne der Muster 5–8 werden von anfangs Blau nach Rot verschoben.

Zur Prüfung der Lichtechtheit der Azofarbstoffe im Leuchtstoffröhren-Testgerät wird wie im Beispiel 1 verfahren.

Die während der Bestrahlungszeit von 96 Stunden sich verändernden optischen Dichten ($D_{ANF \rightarrow END}$) sind in der folgenden Tabelle zusammengestellt.

Tabelle 4

| Probe Nr. | visuelle Dichte $D_{ANF}$ | $D_{END}$ | Rest-dichte % | UV-Dichte $D_{ANF}$ | $D_{END}$ | Rest-dichte % |
|---|---|---|---|---|---|---|
| 5 | 2,21 | 1,01 | 45 | 0,73 | 0,47 | 64 |
| 6 | 1,85 | 0,89 | 48 | 0,86 | 0,59 | 69 |
| 7 | 2,28 | 1,55 | 68 | 0,87 | 0,70 | 81 |
| 8 | 1,85 | 0,96 | 52 | 0,77 | 0,57 | 74 |
| 9 | 1,90 | 1,56 | 82 | 0,76 | 0,67 | 88 |

Die Dichtewerte zeigen, dass der Azofarbstoff des Volltonmusters 9 durch die Emission der Leuchtstoffröhren am wenigsten zerstört wurde.

Beispiel 3

Ausgehend von jeweils 100 ml Baislack werden 4 verschiedene Beschichtungslösungen hergestellt.:

360 mg Sulfosalicylsäure

420 mg 2,5-Dibutoxy-4-morpholinobenzoldiazoniumfluoborat

1 × $10^{-3}$ Mol Blaukuppler

Folgende Blaukuppler wurden verwendet:

10.: 2-Hydroxy-3-naphthoesäure-N(phenyl)-amid-6-sulfonsäureamid (Nr. 10 der Formeltabelle)

11.: 2-Hydroxy-3-naphthoesäure-N(phenyl)-amid-6-sulfonsäure-N(2-methoxyäthyl)-amid (Nr. 12)

12.: 2-Hydroxy-3-naphthoesäure-N(phenyl)-amid-6-sulfonsäuremorpholid (Nr. 25)

13.: 2-Hydroxy-3-naphtoesäure-N(phenyl)-amid-6-sulfon-N(methyl-phenyl)-amid (Nr. 24)

Von jeder Lösung wird, wie im Beispiel 1 beschrieben, ein Diazotypiematerial hergestellt, entsprechend der verwendeten Lösungen fortlaufend von 10–13 numeriert und entwickelt. Nach dem Auslüften und Ausbelichten der so erhaltenen Volltonmuster wird zur Beurteilung der Lichtechtheit ihrer Azofarbstoffe wie in den Beispielen 1 und 2 verfahren.

Die Diazotypiematerialien 10–13 entwickeln zu grünstichig blauen Farbtönen.

Zur Beurteilung der Lichtechtheit der Volltonmuster 10–13 wurden ihre optischen Dichten (D) zu Beginn (ANF) des Bestrahlungsversuches und nach 18 Stunden Standzeit (END) im Lesegerät in der folgenden Tabelle zusammengestellt.

Tabelle 5

| Probe Nr. | visuelle Dichte $D_{ANF}$ | $D_{END}$ | Rest-dichte % | UV-Dichte $D_{ANF}$ | $D_{END}$ | Rest-dichte % |
|---|---|---|---|---|---|---|
| 10 | 1,69 | 1,06 | 63 | 0,85 | 0,63 | 74 |
| 11 | 2,45 | 1,64 | 67 | 1,04 | 0,81 | 78 |
| 12 | 2,38 | 1,67 | 73 | 1,01 | 0,82 | 81 |
| 13 | 1,94 | 1,40 | 72 | 0,92 | 0,74 | 81 |

Die gemessenen Dichtewerte zeigen, dass der Dichteverlust der Volltonmuster 10–13 im visuellen und ultravioletten Spektralbereich nach 18 Stunden Standzeit im Lesegerät nahezu gleichgross ist. Die Azofarbstoffe der Volltonmuster 12 und 13 sind etwas lichtstabiler als die Azofarbstoffe der Volltonmuster 10 und 11.

Ein Vergleich mit den Dichtewerten der Volltonmuster 1–3 der Tabelle 1 nach 18 Stunden Standzeit im Lesegerät zeigt die überragende Lichtstabilität der Azofarbstoffe der Volltonmuster 10–13.

Der Farbton des Volltonmusters 10 wurde nach 18 Stunden Standzeit im Lesegerät von einem anfangs grünstichig blauen nach einem rotstichig blauen Farbton verschoben.

Zur Beurteilung der Lichtechtheit der Azofarbstoffe im Leuchtstoffröhren-Testgerät wird wie in den Beispielen 1 und 2 verfahren.

Die sich verändernden optischen Dichten (D) der Azofarbstoffe während der Bestrahlungszeit von 96 Stunden sind in der folgenden Tabelle 6 zusammengestellt.

Tabelle 6

| Probe Nr. | visuelle Dichte $D_{ANF}$ | $D_{END}$ | Rest-dichte % | UV-Dichte $D_{ANF}$ | $D_{END}$ | Rest-dichte % |
|---|---|---|---|---|---|---|
| 10 | 1,52 | 1,28 | 84 | 0,78 | 0,71 | 91 |
| 11 | 2,25 | 2,11 | 94 | 0,98 | 0,93 | 95 |
| 12 | 2,02 | 1,88 | 93 | 0,93 | 0,91 | 95 |
| 13 | 1,77 | 1,62 | 92 | 0,85 | 0,81 | 95 |

Die Dichtewerte zeigen, dass der Azofarbstoff des Volltonmusters 10 etwas weniger lichtecht ist als die Azofarbstoffe der Volltonmuster 11–13.

Ein Vergleich mit den Dichtewerten der Volltonmuster 4 und 9 der Beispiele 1 bzw. 2 zeigt aber auch, dass der Dichteabfall während der Bestrahlungszeit von 96 Stunden im Leuchtstoffröhren-Testgerät praktisch gleichgross ist.

Die Messergebnisse zeigen ausserdem, dass eine sekundäre und tertiäre Sulfonsäureamidgruppe in 6-Position des 2-Hydroxy-3-naphthoesäureamid-Moleküls die Lichtechtheit der mit diesen Verbindungen erzielbaren Azofarbstoffe günstiger beeinflusst als eine primäre Sulfonsäureamidgruppe in der gleichen Position.

Beispiel 4

Ausgehend von jeweils 100 ml Basislack wurden 5 verschiedene Beschichtungslösungen hergestellt mit:

744 mg Sulfosalicylsäure
153 mg Zinkchlorid
600 ml Glycerintriacetat
168 mg Thioharnstoff
153 mg 2-Methylresorcin
30 mg 2,2',4,4'-Tetrahydroxy-diphenylsulfid
660 mg 2,5-Dibutoxy-4-morpholino-benzoldiazoniumfluaborat
70 mg 4-(Dipropylamino)-benzoldiazoniumfluoborat
2 × $10^{-3}$ Mol Blaukuppler
wobei als Blaukuppler eingesetzt wurden:
14.: 2-Hydroxy-3-naphthoesäure-N(2-methylphenyl)-amid-6-sulfonsäure-N(phenyl)-amid (Nr. 16)
15.: 2-Hydroxy-3-naphthoesäure-N(phenyl)-amid-6-sulfonsäure-N(phenyl)-amid (Nr. 15)
16.: 6-Methoxy-2-hydroxy-3-naphthoesäure-N(3morpholino-propyl)-amid
17.: 2-Hydroxy-3-naphtohesäure-N(3-morpholino-propyl)-amid
18.: 2-Hydroxy-3-naphthoesäure-N(2-methylphenyl)-amid

Jede der Lösungen wird, wie im Beispiel 1 beschrieben, auf eine mit einer Haftschicht versehene Polyäthylenterephthalatfolie von 100 µm Dicke aufgetragen, deren Rückseite eine mit Siliziumdioxid und Aluminiumoxid pigmentierte Lackschicht trägt. Die Sensibilisierungslösung wird dabei so aufgebracht, dass nach dem Trocknen die verschiedenen Diazotypiematerialien zu einer optischen visuellen Dichte (D) von 1,5–2,5 entwickelt werden können.

Die entwickelte Volltonmuster werden entsprechend der verwendeten Beschichtungslösungen fortlaufend von 14–18 numeriert.

Die Diazotypiematerialien 14 und 15 entwickeln zu einem neutral schwarzen, das Diazotypiematerial 16 zu einem grünstichig dunkelblauen, das Diazotypiematerial 17 zu einem violetten und das Diazotypiematerial 18 zu einem rotstichig blauen Farbton.

Von jedem Volltonmuster wird, wie in Beispiel 1 beschrieben, ein Probestreifen abgeschnitten und mit der spektralen Emission der Lichtquelle des Lesegerätes bestrahlt.

Zur Beurteilung der Lichtechtheit der Azofarbstoffe der Volltonmuster 14–18 sind ihre optischen Dichten zu Beginn (ANF) des Bestrahlungsversuches und nach 10 Stunden Standzeit (END) im Lesegerät in der folgenden Tabelle zusammengestellt.

Tabelle 7

| Probe Nr. | visuelle Dichte $D_{ANF}$ | $D_{END}$ | Restdichte % | UV-Dichte $D_{ANF}$ | $D_{END}$ | Restdichte % |
|---|---|---|---|---|---|---|
| 14 | 1,66 | 1,35 | 82 | 1,25 | 1,15 | 92 |
| 15 | 1,74 | 1,45 | 83 | 1,34 | 1,23 | 92 |
| 16 | 2,14 | 1,24 | 58 | 1,43 | 1,27 | 89 |
| 17 | 2,34 | 1,22 | 52 | 1,16 | 0,97 | 83 |
| 18 | 2,55 | 1,44 | 56 | 1,21 | 1,02 | 85 |

Ein Vergleich der gemessenen optischen Dichten zeigt, dass die Azofarbstoffe der Volltonmuster 14 und 15 nach 10 Stunden Standzeit im Lesegerät besonders im visuellen Spektralbereich erheblich lichtstabiler sind als die Azofarbstoffe der Volltonmuster 16–18.

Zu ähnlichen Ergebnissen in bezug auf Lichtechtheit gelangt man, wenn man anstelle der einseitig pigmentierten Polyäthylenterephthalatfolie ein in der Diazotypie gebräuchliches natürliches Transparentpapier verwendet.

Beispiel 5

Eine mit einer Haftschicht versehene Polyäthylenterephthalatfolie wird einseitig auf der substrierten Oberfläche mit einer Lösung folgender Zusammensetzung beschichtet:
100 ml Basislack
360 mg Sulfosalicylsäure
10 mg Thioharnstoff
420 mg 2-Hydroxy-3-naphtoesäure-N(phenyl)-amid-6-sulfonsäure-N(phenyl)-amid (Nr. 15)
410 mg 6-Chlor-4-dimethylamino-3(4-chlor-phenoxy)-benzoldiazoniumchlorid (Zinkchlorid-Doppelsalz)

Nach dem Trocknen der sensibilisierten Folie wird, wie üblich, mit feuchtem Ammoniakgas entwickelt. Das Diazotypiematerial entwickelt nach einmaligem Durchlauf durch den Entwicklerteil eines handelsüblichen Dupliziergerätes zu einem kräftigen, rotstichig blauen Farbton, dessen visuelle Dichte (D) sich auch bei wiederholter Entwicklung nicht ändert. Das unbelichtete Diazotypiematerial ist unter tropischen klimatischen Bedingungen ausreichend haltbar.

Beispiel 6

Ein in der Diazotypie übliches Lichtpausrohpapier mit einem einseitigen Vorstrich aus kolloidaler Kieselsäure und Polyvinylacetat wurde auf der vorbehandelten Oberfläche mit einer Lösung folgender Zusammensetzung bestrichen:
100 ml Wasser
4 g Zitronensäure
5 g Thioharnstoff
2,3 g 2-Hydroxy-3-naphthoesäure-N(3-morpholino-propyl)-amid-6-sulfonsäure-N(phenyl)-amid (Zinkchlorid-Komplexsalz) (Nr. 4)
1,5 g 2,5-Diäthoxy-4-morpholino-benzoldiazoniumchlorid (Zinkchlorid-Doppelsalz)

Nach dem Trocknen wurde das sensibilisierte Papier unter einer transparenten Vorlage bildmässig belichtet und anschliessend die latente Kopie mit feuchtem Ammoniakgas in einem handelsüblichen Lichtpausgerät entwickelt. Es wurde ein grünstichig blaues Bild auf weissem Grund erhalten.

Die Haltbarkeit des unbelichteten Diazotypiematerials unter tropischen klimatischen Bedingungen und die Lichtechtheit der blaugrünen Azofarbstoffbilder der Diazotypiekopie im Leuchtstoffröhren-Testgerät sind sehr gut.

Ersetzt man den verwendeten erfindungsgemässen Kuppler durch einen üblichen Blaukuppler, wie 1,4 g 2-Hydroxy-3-naphthoesäure-N(3-morpholinopropyl)-amid (Hydrochlorid), so erhält man nach der bildmässigen Belichtung und anschliessend Entwicklung ein rotstichig blaues Bild auf schwach beigem Grund. Die Haltbarkeit dieses unbelichteten Diazotypiematerials und die Lichtechtheit des Azofarbstoffbildes sind schlechter als bei Verwendung des erfindungsgemässen Blaukupplers.

Beispiel 7

Weisses Lichtpausrohpapier, das einseitig mit einem Vorstrich aus kolloidaler Kieselsäure und Polyvinylacetat versehen ist, wird auf der vorbehandelten Oberfläche mit einer Lösung folgender Zusammensetzung bestrichen:

100 ml Wasser
0,5 g Weinsäure
0,1 g Saponin
1,6 g Zinkchlorid-Doppelsalz von 2,5-Diäthoxy-4(4'methyl-phenylmercapto)-benzoldiazoniumchlorid

Nach dem Trocknen wurde das sensibilisierte Papier unter einer transparenten Vorlage bildmässig belichtet und anschliessend die latente Kopie mit einer Lösung der folgenden Zusammensetzung entwickelt:

100 ml Wasser
1,0 g Kaliumhydroxid
0,1 g Na-Salz einer Alkylnaphthalin-sulfonsäure
1,5 g 2-Hydroxy-3-naphthoesäure-N(phenyl)-amid-6-sulfonsäure-N(3'-methoxypropyl)-amid (Nr. 13)

Man erhält eine Kopie der Vorlage mit neutral blauen Linien mit sehr guter Lichtechtheit des Azofarbstoffbildes im Leuchtstoffröhren-Testgerät.

| Nr. | $R_1$ | $R_2$ | $R_3$ | Fp. (°C) | Herst.-verf. |
|---|---|---|---|---|---|
| 1 | H | 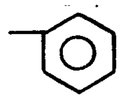 | H | 233–235 | A |
| 2 | $-(CH_2)_2-OH$ | 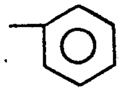 | H | 187–188 | A |
| 3 | 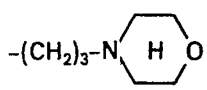 | 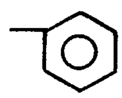 | H | 163–165 | A |
| 4 | 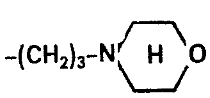 · $ZnCl_2$ HCl | 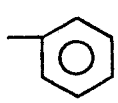 | H | 124 (Z.) | A |
| 5 | 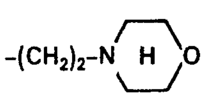 · $HPF_6$ | 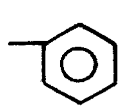 | H | 100 (Z.) | A |
| 6 | $-(CH_2)_2-N(C_2H_5)_2$ | 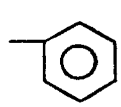 | H | 107–110 | A |
| 7 | 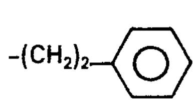 | 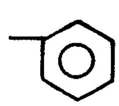 | H | 207–209 | A |
| 8 | 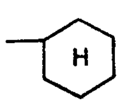 | 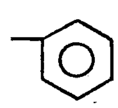 | H | 223–225 | B |

| Nr. | $R_1$ | $R_2$ | $R_3$ | Fp. (°C) | Herst.-verf. |
|---|---|---|---|---|---|
| 9 | $(CH_2)_3$– 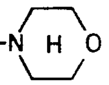 · HCl | $-C_2H_5$ | $-C_2H_5$ | 214–216 | A |
| 10 | 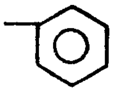 | H | H | 274 (Z.) | B |
| 11 | 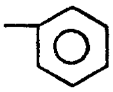 | $-C_4H_9(n)$ | H | 208–209 | B |
| 12 | 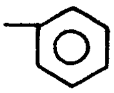 | $-(CH_2)_2-OCH_3$ | H | 210–213 | B |
| 13 | 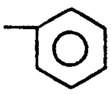 | $-(CH_2)_3-OCH_3$ | H | 176–177 | B |
| 14 | 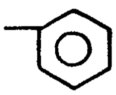 | $-CH_2$– 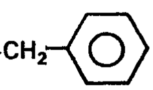 | H | 234–236 | B |
| 15 | 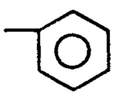 | 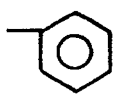 | H | 241–243 | B |
| 16 | 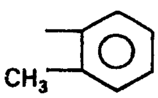 | 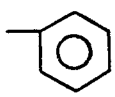 | H | 232–233 | B |
| 17 | 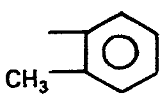 | 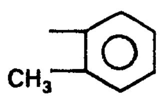 | H | 228–229 | B |
| 18 | 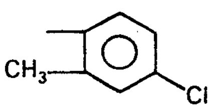 | 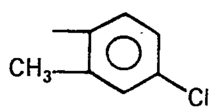 | H | 250–251 | B |
| 19 | 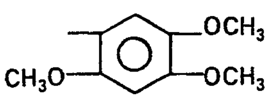 | 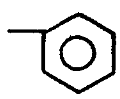 | H | 252–253 | B |
| 20 | 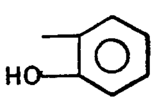 | 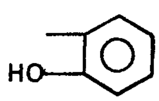 | H | 253–254 | B |
| 21 | 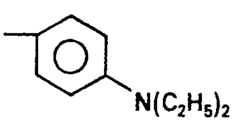 | $-(CH_2)_2-OCH_3$ | H | 207–208 | B |
| 22 | 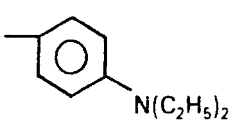 · HCl | $-(CH_2)_2-OCH_3$ | H | 241–243 (Z.) | B |

| Nr. | $R_1$ | $R_2$ | $R_3$ | Fp. (°C) | Herst.-verf. |
|---|---|---|---|---|---|
| 23 | 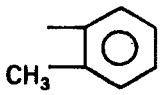 | $-C_2H_5$ | $-C_2H_5$ | 242–243 | B |
| 24 | 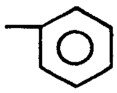 | $-CH_3$ | 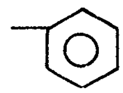 | 221–223 | B |
| 25 | 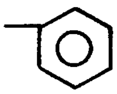 | 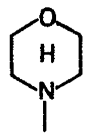 | | 244–245 | B |
| 26 | 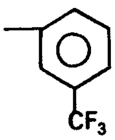 | 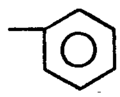 | H | 257–259 | B |
| 27 | 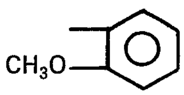 | 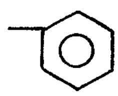 | H | 225–227 | B |
| 28 | 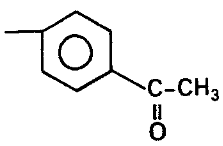 | 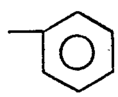 | H | 264–265 | B |
| 29 | 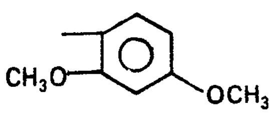 | 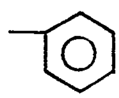 | H | 214–215 | B |
| 30 | 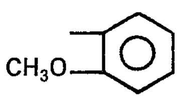 | $-(CH_2)_3-OCH_3$ | H | 156–157 | B |
| 31 | 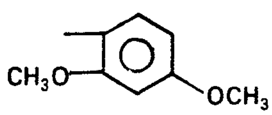 | $-(CH_2)_3-OCH_3$ | H | 151–153 | B |
| 32 | 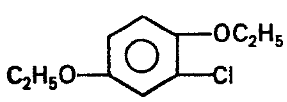 | $-(CH_2)_3-OCH_3$ | H | 162–163 | B |
| 33 | 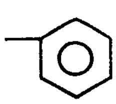 | $-CH_3$ | H | 244–245 | B |
| 34 | $-CH_3$ | $-CH_3$ | H | 248–249 | A |
| 35 | 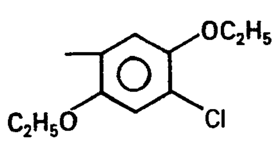 | 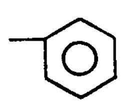 | H | 237–239 | B |

| Nr. | $R_1$ | $R_2$ | $R_3$ | Fp. (°C) | Herst.-verf. |
|---|---|---|---|---|---|
| 36 | 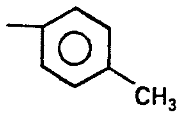 | 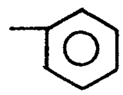 | H | 249–250 | B |
| 37 | 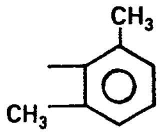 | 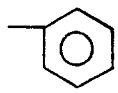 | H | 233–236 | B |
| 38 | 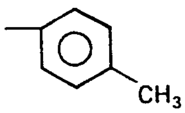 | 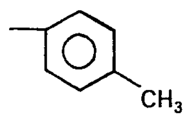 | H | 263–264 | B |
| 39 | 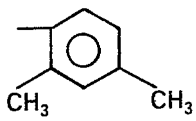 | 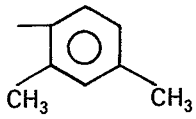 | H | 219–222 | B |
| 40 | 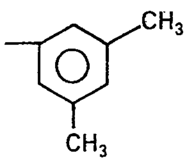 | 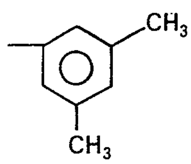 | H | 220–223 | B |
| 41 | 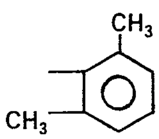 | 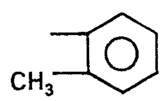 | H | 211–213 | B |
| 42 | 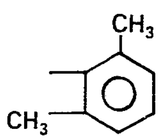 | 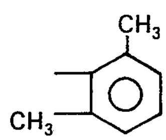 | H | 268–273 | B |

**Patentansprüche**

1. 2-Hydroxy-3-naphthoesäureamide der allgemeinen Formel

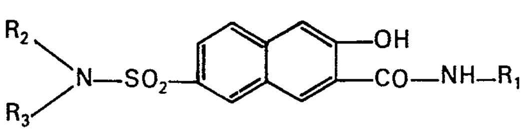

worin $R_1$ Wasserstoff oder Alkyl, Cycloalkyl, Aralkyl, Aryl, welche durch Hydroxyl, Alkyl, Halogenalkyl, Alkoxyl, Acyl, Halogen, Dialkylamino oder einen heterocyclischen Rest substituiert sein können, und
$R_2$ und $R_3$ gleich oder verschieden sind und Wasserstoff oder Alkyl, Cycloalkyl, Aralkyl, Aryl, welche durch Hydroxyl, Niederalkyl, Alkoxyl, Acyl oder Halogen substituiert sein können, oder zusammen mit dem Stickstoffatom, an das sie gebunden sind, einen heterocyclischen Rest, der durch Alkyl substituiert sein kann,
bedeuten.

2. 2-Hydroxy-3-naphthoesäureamide nach Anspruch 1, worin $R_1$ und $R_2$ gleiches oder verschiedenes Aryl, welches durch Hydroxyl, Niederalkyl, Alkoxyl oder Halogen substituiert sein kann, und
$R_3$ Wasserstoff bedeuten.

3. Naphthoesäureamide der allgemeinen Formel nach Anspruch 1, worin $R_1$ Wasserstoff, Alkyl mit bis zu 4 Kohlenstoffatomen, Cycloalkyl mit bis zu 12 Kohlenstoffatomen, Aralkyl mit bis zu 10 Kohlenstoffatomen, oder Aryl mit bis zu 10 Kohlenstoffatomen, welche durch Hydroxyl, gegebenenfalls halogensubstituiertes Alkyl, Alkoxyl, Acyl, Halogen, durch gegebenenfalls Alkyl-substituierte Aminogruppen oder durch einen heterocyclischen Rest substituiert sein können, $R_2$ und $R_3$ gleich oder verschieden sind, und Wasserstoff, Alkyl mit bis zu 6 Kohlenstoffatomen, Cycloalkyl mit bis zu 6 Kohlenstoffatomen, Aralkyl mit bis zu 10 Kohlenstoffatomen oder Aryl mit bis zu 10 Kohlenstoffatomen, welche durch Hydroxyl, niederes Alkyl, Alkoxyl oder Acyl, Halogen substituiert sein können, oder zusammen mit dem Stickstoffatom, an das sie gebunden sind, gegebenenfalls Alkyl-substituiertes Pyrrolidin, Piperidin, Piperazin, Thiomorpholin, Morpholin oder Hexamethylenimin bedeuten.

4. Napthoesäureamide nach Ansprüchen 1 bis 3, worin $R_1$ und $R_2$ Phenyl, das durch mindestens ein Hydroxyl, niederes Alkyl oder Alkoxyl oder Halogen substituiert sein kann, und $R_3$ Wasserstoff bedeuten.

5. Naphthoesäureamide nach Ansprüchen 1 und 3, worin $R_1$ und/oder $R_2$ durch mindestens einen basischen Rest substituiert sind.

6. Naphthoesäureamid nach Ansprüchen 1 bis 5 als Kupplungskomponenten in Diazotypiematerialien.

**Claims**

1. 2-hydroxy-3-naphthoic acid amides of the general formula

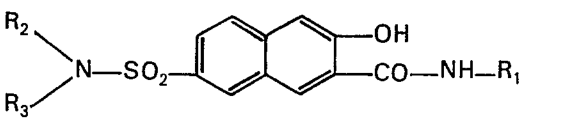

wherein $R_1$ is hydrogen or an alkyl, cycloalkyl, aralkyl or aryl group, which may be substituted by a hydroxy, alkyl, halogenalkyl, alkoxy or acyl group, a halogen atom, a dialkylamino group or a heterocyclic group, and
$R_2$ and $R_3$ are identical or different and are hydrogen or alkyl, cycloalkyl, aralkyl or aryl groups, which may be substituted by a hydroxy, lower alkyl, alkoxy or acyl group or by a halogen atom or, together with the nitrogen atom to which they are attached, a heterocyclic group which may be substituted by an alkyl group.

2. 2-hydroxy-3-naphthoic acid amides as claimed in claim 1, wherein $R_1$ and $R_2$ are identical or different aryl groups which may be substituted by a hydroxy, lower alkyl or alkoxy group or by a halogen atom and $R_3$ is hydrogen.

3. Naphthoic acid amides of the general formula as claimed in claim 1, wherein $R_1$ is hydrogen, an alkyl group containing up to 4 carbon atoms, a cycloalkyl group containing up to 12 carbon atoms, an aralkyl group containing up to 10 carbon atoms, or an aryl group containing up to 10 carbon atoms, which may be substituted by a hydroxy group, an alkyl group which may be substituted by halogen atoms, an alkoxy group, an acyl group, a halogen atom, by amino groups which may be substituted by alkyl groups, or by a heterocyclic group; in which $R_2$ and $R_3$ are identical or different and represent hydrogen, an alkyl group containing up to 6 carbon atoms, a cycloalkyl group containing up to 6 carbon atoms, an aralkyl group containing up to 10 carbon atoms, or an aryl group containing up to 10 carbon atoms, which may be substituted by a hydroxy, a lower alkyl, an alkoxy, or an acyl group, or by a halogen atom or which, together with the nitrogen atom to which they are attached, represent a pyrrolidone, a piperidine, a piperazine, a thiomorpholine, a morpholine, or a hexamethyleneimine ring which may be substituted by an alkyl group.

4. Naphthoic acid amides as claimed in claims 1 to 3, wherein $R_1$ and $R_2$ are phenyl groups which may be substituted by at least one hydroxy, lower alkyl or alkoxy group or by a halogen atom, and wherein $R_3$ is hydrogen.

5. Naphtoic acid amides as claimed in claims 1 and 3, wherein $R_1$ and/or $R_2$ are substituted by at least one basic group.

6. Napthoic acid amide as claimed in claims 1 to 5, as a coupling component in diazotype materials.

**Revendications**

1. 2-hydroxy-3-naphtamides de la formule générale:

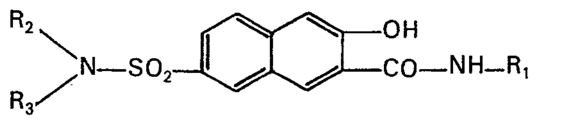

dans laquelle $R_1$ représente un atome d'hydrogène ou un groupe alkyle, cyclo-alkyle, aryle, pouvant être substitués par des groupes hydroxyle, alkyle, halogéno-alkyle, alcoxyle, acyle, des halogènes, des groupes dialkylamine ou un radical hétérocyclique, et
$R_2$ et $R_3$ sont semblables ou différents et représentent un atome d'hydrogène ou un groupe alkyle, cyclo-alkyle, aralkyle, aryle pouvant être substitués par des groupes hydroxyle, alkyle inférieur, alcoxyle, acyle ou des halogènes, ou bien représentent, avec l'atome d'azote auquel ils sont rattachés, un radical hétérocyclique qui peut être substitué par un groupe alkyle.

2. 2-hydroxy-3-naphtamides selon la revendication 1, caractérisés en ce que $R_1$ et $R_2$ représentent un groupe aryle semblable ou différent qui peut être substitué par des groupes hydroxyle, alkyle inférieur, alcoxyle ou des halogènes et $R_3$ un atome d'hydrogène.

3. Naphtamides de la formule générale selon la revendication 1, caractérisés en ce que $R_1$ représente un atome d'hydrogène, un groupe alkyle contenant jusqu'à 4 atomes de carbone, un groupe cyclo-alkyle contenant jusqu'à 12 atomes de carbone, un groupe aralkyle contenant jusqu'à 10 atomes de carbone ou un groupe aryle contenant jusqu'à 10 atomes de carbone, pouvant être substitués par des groupes hydroxyle, des groupes alkyle éventuellement halogénées, des groupes alcoxyle, acyle, des halogènes, des groupes amine éventuellement alkylés ou un radical hétérocyclique, $R_2$ et $R_3$ sont semblables ou différents et représentent un atome d'hydrogène, un groupe alkyle contenant jusqu'à 6 atomes de carbone, un groupe cyclo-alkyle contenant jusqu'à 6 atomes de carbone, un groupe aralkyle contenant jusqu'à 10 atomes de carbone ou un groupe aryle contenant jusqu'à 10 atomes de carbone, pouvant être substituées par des groupes hydroxyle, alkyle inférieur, alcoxyle ou acyle ou des halogènes, ou bien représentent, avec l'atome d'azote auquel ils sont rattachés, un radical pyrrolidine, pipéridine, pipérazine, thiomorpholi-

ne, morpholine ou hexaméthylène-imine, éventuellement alkylés.

4. Naphtamides selon les revendications 1 à 3, caractérisés en ce que $R_1$ et $R_2$ représentent un groupe phényle qui peut être substitué par au moins un groupe hydroxyle, alkyle inférieur ou alcoxyle ou des halogènes et $R_3$ un atome d'hydrogène.

5. Naphtamides selon les revendications 1 et 3, caractérisés en ce qu $R_1$ et/ou $R_2$ sont substituées par au moins un radical basique.

6. Naphtamide selon les revendications 1 à 5, en tant que couplant dans des matériaux de diazotypie.

FIG. 1

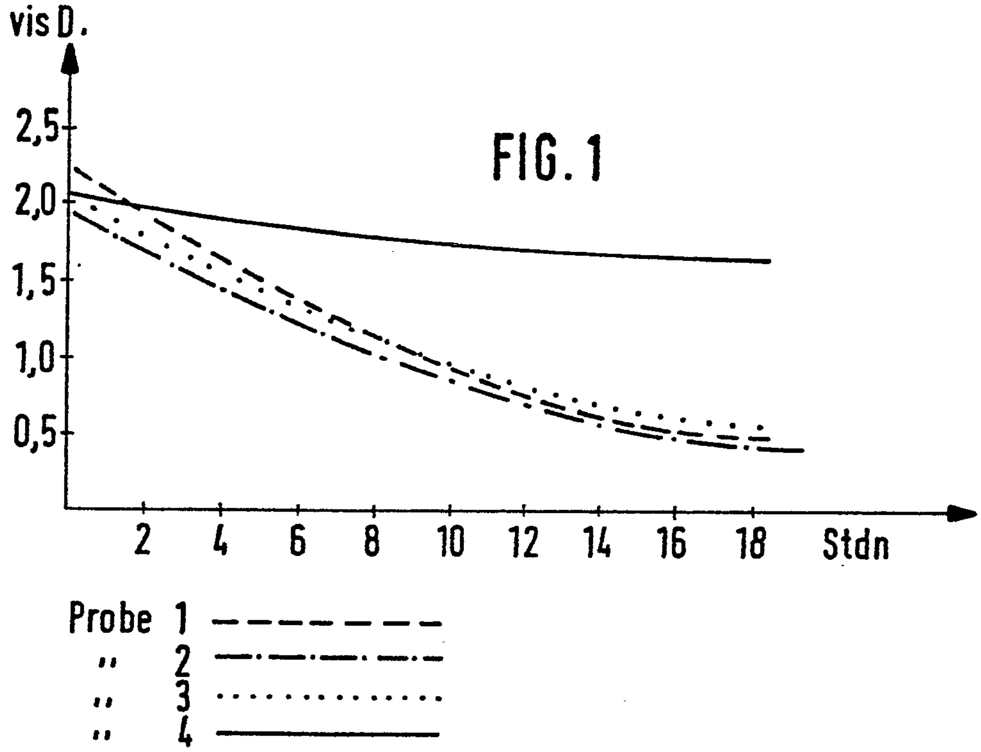
vis D.
2,5
2,0
1,5
1,0
0,5
2
4
6
8
10
12
14
16
18
Stdn
Probe 1
" 2
" 3
" 4

FIG. 2

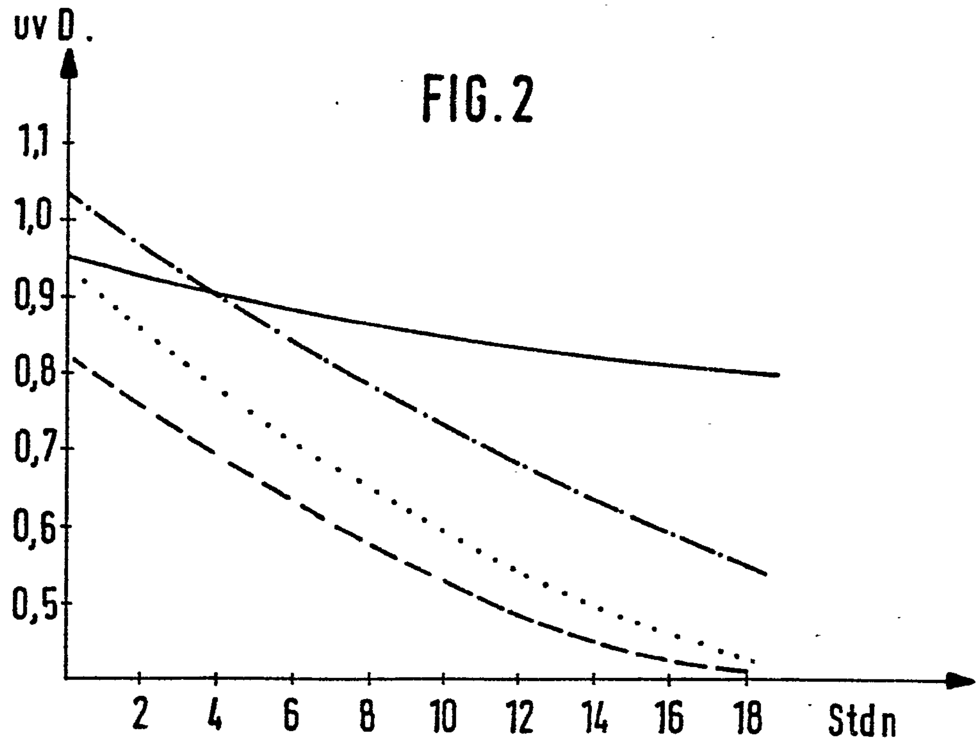
uv D.
1,1
1,0
0,9
0,8
0,7
0,6
0,5
2
4
6
8
10
12
14
16
18
Stdn